# EUROPEAN PATENT APPLICATION

(11) **EP 3 822 356 A1**
(43) Date of publication of application: **19.05.2021**
(21) Application number: 19209853.1
(22) Date of filing: 18.11.2019
(51) Int. Cl.: C12P 7/44, C07C 51/42, C07C 51/43, C07C 51/44, B01D 61/14

(54) **METHOD FOR THE PRODUCTION OF A DICARBOXYLIC ACID**

(71) Applicant: Corvay Bioproducts GmbH, 06237 Leuna (DE)
(72) Inventor: The designation of the inventor has not yet been filed
(74) Representative: Bohmann, Armin K.

(57) **Abstract**

The present invention is related to a method for the production of a dicarboxylic acid, wherein the method comprises
- a bioconversion step, wherein in the bioconversion step, the dicarboxylic acid is produced from a precursor compound contained in a medium; and a
- purification step for purifying the dicarboxylic acid from the medium, wherein the purification step comprises a nano-diafiltration step and/or a distillation step, wherein preferably if the purification step comprises both the nano-diafiltration step and the distillation step, the nano-diafiltration step is carried out prior to the distillation step, and
wherein the dicarboxylic acid is selected from the group comprising decanedioic acid, dodecanedioic acid, tetradecanedioic acid and hexadecanedioic acid, preferably the dicarboxylic acid is dodecanedioic acid (DDDA).

## Description

The present invention is related to a method for the production of a dicarboxylic acid, a dicarboxylic acid produced by such method, the use of nanofiltration or a nanofiltration device in a or the method for the production and/or purification of the dicarboxylic acid, the use of distillation in a or the method for the production and/or purification of the dicarboxylic acid, and the use of a thin-film evaporator in a or the method for the production and/or purification of the dicarboxylic acid.

Dicarboxylic acids are used in the preparation of polymers such as polyamides and polyesters. Representative dicarboxylic acids include, but are not limited to decanedioic acid, dodecanedioic acid, tetradecanedioic acid and hexadecanedioic acid.

Dodecanedioic acid (DDDA) is a dicarboxylic acid mainly used in hot melt adhesives, top-grade coatings, painting materials, corrosion inhibitors, lubricants, and engineering plastics such as nylon 612, nylon 1212 and nylon 1012. Experimental work with dodecanedioic acid in type 2 diabetic patients has demonstrated that IV infusion helps to maintain normal blood sugar and energy levels without increasing the blood glucose load in the process.

DDDA is currently produced by both chemical and biological processes.

The chemical process uses butadiene as a starting material in multi-step chemical process. Butadiene is first converted to cyclododecatriene through a cyclotrimerization process. Cyclododecatriene is hydrogenated to cyclododecane followed by air oxidation in the presence of boric acid at elevated temperatures to a mixture of an alcohol derivative and a ketone derivative of the cyclododecane. In the final step, this mixture oxidized further by nitric acid to produce DDDA.

In the biological process, paraffin oil mainly containing dodecane is converted into DDDA with particular strains of Candida tropicalis yeast in a multi-step process. Renewable plant-oil feedstocks sourced can also be used to as a starting material in such biological process.

The problem underlying the present invention is the provision of a biotechnological process for the production of a dicarboxylic acid such as decanedioic acid, dodecanedioic acid, tetradecanedioic acid and hexadecanedioic acid.

It is a further problem underlying the present invention to provide a method for the production of a dicarboxylic acid such as decanedioic acid, dodecanedioic acid, tetradecanedioic acid and hexadecanedioic acid, whereby such process provides for an increased purity of such dicarboxylic acid.

It is still a further problem underlying the present invention to provide means for the production of a dicarboxylic acid such as decanedioic acid, dodecanedioic acid, tetradecanedioic acid and hexadecanedioic acid, preferably by a biotechnological process, wherein the produced dicarboxylic acid shows increased purity.

Another problem underlying the present invention is the provision of a means for the purification of dicarboxylic acid such as decanedioic acid, dodecanedioic acid, tetradecanedioic acid and hexadecanedioic acid, preferably from a medium containing the dicarboxylic acid, whereby more preferably the medium is the result of a bioconversion step which is most preferably free of any cellular material or debris of such cellular material.

Still another problem underlying the present invention is the provision of a method for the purification of dicarboxylic acid such as decanedioic acid, dodecanedioic acid, tetradecanedioic acid and hexadecanedioic acid, preferably from a medium containing the dicarboxylic acid, whereby more preferably the medium is the result of a bioconversion step which is most preferably free of any cellular material or debris of such cellular material.

These and other problems underlying the present invention are solved by the subject matter of the attached independent claims. Preferred embodiments may be taken from the attached dependent claims.

More specifically, the problem underlying the present invention is solved in a first aspect by a method for the production of a dicarboxylic acid, wherein the method comprises
- a bioconversion step, wherein in the bioconversion step, the dicarboxylic acid is produced from a precursor compound contained in a medium; and a
- purification step, wherein the purification step comprises a nano-diafiltration step, wherein the medium containing the dicarboxylic acid is subjected to a nano-diafiltration step, wherein the retentate of the nano-diafiltration contains the dicarboxylic acid.

More specifically, the problem underlying the present invention is solved in a second aspect by a method for the production of a dicarboxylic acid, wherein the method comprises
- a bioconversion step, wherein in the bioconversion step, the dicarboxylic acid is produced from a precursor compound contained in a medium; and
- a purification step, wherein the purification step comprises a distillation step, wherein in the distillation step, the dicarboxylic acid obtained from the bioconversion step acid is subjected to distillation.

More specifically, the problem underlying the present invention is solved in a third aspect by the dicarboxylic acid obtained or obtainable by the method according to the first aspect, including any embodiment thereof.

Furthermore, the problem underlying the present invention is solved in a fourth aspect by the use of nano-diafiltration device in a method for producing a dicarboxylic acid, preferably in a method according to the first aspect and the second aspect, including any embodiment thereof.

The problem underlying the present invention is solved in a fifth aspect by the use of nano-diafiltration in a method for producing a dicarboxylic acid, preferably the method is a method of purifying a dicarboxylic acid.

The problem underlying the present invention is solved in a sixth aspect by a distillation step in a method of producing and/or purifying a dicarboxylic acid, preferably the method is a method of purifying a dicarboxylic acid.

The problem underlying the present invention is solved in a seventh aspect by the use of a thin-film evaporator, preferably a thin-fil evaporator in a method of producing and/or purifying a dicarboxylic acid, preferably the method is a method of purifying a dicarboxylic acid.

The present invention is also solved by the following embodiments 1 to 125, whereby embodiment 1 corresponds to the first aspect, embodiment 61 corresponds to the second aspect, embodiment 109 corresponds to the third aspect, embodiment 110 corresponds to the fourth aspect, embodiment 112 corresponds to the fifth aspect, embodiment 115 corresponds to the sixth aspect, and embodiment 117 corresponds to the seventh aspect.
Embodiment 1: A method for the production of a dicarboxylic acid, wherein the method comprises
   - a bioconversion step, wherein in the bioconversion step, the dicarboxylic acid is produced from a precursor compound contained in a medium; and a
   - purification step, wherein the purification step comprises a nano-diafiltration step, wherein the medium containing the dicarboxylic acid is subjected to a nano-diafiltration step, wherein the retentate of the nano-diafiltration contains the dicarboxylic acid.
Embodiment 2. The method of Embodiment 1, wherein the membrane used in the nano-diafiltration step has a cut-off value of between 150 Da and 300 Da, preferably the cut-off value is ≤ 150 Da.
Embodiment 3: The method of any one of Embodiments 1 to 2, wherein the dicarboxylic acid containing retentate of the nano-diafiltration step is subjected to an acidification step.
Embodiment 4. The method of Embodiment 3, wherein in the acidification step, sulfuric acid is added to the dicarboxylic acid containing retentate of the nano-diafiltration step and the dicarboxylic acid is precipitated from the retentate, and the precipitated dicarboxylic acid is optionally washed.
Embodiment 5. The method of Embodiment 4, wherein the precipitated dicarboxylic acid is subjected to a distillation step, wherein in the distillation step, the precipitated dicarboxylic acid is melted, preferably at a temperature of about 140 °C, and subjected to distillation.
Embodiment 6. The method of Embodiment 5, wherein in the distillation step, the melted dicarboxylic acid is heated so as to obtain vaporized dicarboxylic acid, preferably the melted dicarboxylic acid is heated in a distillation column so as to obtain vaporized dicarboxylic acid.
Embodiment 7. The method of Embodiment 6, wherein in the distillation column vaporized dicarboxylic acid is separated from a high-boiler and/or a low-boiler, preferably a high-boiler and/or a low-boiler comprised in or associated with the precipitated dicarboxylic acid.
Embodiment 8. The method of any one of Embodiments 6 to 7, wherein the dicarboxylic acid is vaporized at a temperature of about 190 °C to about 240°C.
Embodiment 9. The method of any one of Embodiments 6 to 8, wherein the dicarboxylic acid is vaporized at a pressure of 1 hPa to 10 hPa.
Embodiment 10. The method of any one of Embodiments 8 to 9, wherein the conditions for vaporization of the dicarboxylic acid ranges from 190°C at 1 hPa to about 240°C at 10 hPa.
Embodiment 11. The method of any one of Embodiments 6 to 10, wherein the dicarboxylic acid is vaporized in a thin-film evaporator, wherein in the thin-film evaporator the high-boiler is separated from the dicarboxylic acid.
Embodiment 12. The method of Embodiment 11, wherein the dicarboxylic acid obtained from the thin-film evaporator is conducted to a rectification column, wherein in the rectification column the dicarboxylic acid is separated from the low-boiler.
Embodiment 13. The method of Embodiment 12, wherein the dicarboxylic acid is introduced to a feed tray at the middle section of the rectification column.
Embodiment 14. The method of any one of Embodiments 12 to 13, wherein the rectification column comprises at least eight trays.
Embodiment 15. The method of any one of Embodiments 12 to 14, wherein the dicarboxylic acid is introduced into the rectification column at a temperature of about 190 °C to about 240 °C.
Embodiment 16. The method of any one of Embodiments 12 to 15, wherein the dicarboxylic acid is introduced into the rectification column at a pressure of about 1 hPA to about 10 hPa.
Embodiment 17. The method of any one of Embodiments 12 to 16, wherein the dicarboxylic acid is introduced into the rectification column at temperature/pressure ranges from 190°C at 1 hPa to about 240°C at 10 hPa.
Embodiment 18. The method of any one of Embodiments 12 to 17, wherein the dicarboxylic acid obtained in the distillation step is removed from the bottom of the rectification column.
Embodiment 19. The method of any one of Embodiments 4 to 18, wherein, prior to the acidification step, activated carbon is added to the retentate of the nano-diafiltration step.
Embodiment 20. The method of Embodiment 19, wherein the activated carbon comprising retentate of the nano-diafiltration step is heated, preferably heated to a temperature from about 60 °C to about 90 °C.
Embodiment 21. The method of any one of Embodiments 19 to 20, wherein the activated carbon is removed from the retentate of the nano-diafiltration step and the thus obtained retentate of the nano-diafiltration step is subjected to the acidification step.
Embodiment 22. The method of any one of Embodiments 1 to 4, wherein the precipitated dicarboxylic acid is dissolved in a fluid.
Embodiment 23. The method of Embodiment 22, wherein the fluid is water, an organic solvent or a mixture of water and an organic solvent.
Embodiment 24. The method of Embodiment 23, wherein the organic solvent is acetic acid.
Embodiment 25. The method of any one of Embodiments 22 to 24, wherein activated carbon is added to the dicarboxylic acid containing fluid.
Embodiment 26. The method of Embodiment 25, wherein the fluid containing activated carbon and dicarboxylic acid the is incubated at a temperature of about 90 °C or higher.
Embodiment 27. The method of Embodiment 26, wherein the fluid containing activated carbon and the dicarboxylic acid is kept at a temperature of about 90 °C or higher for 30 minutes to 2 hours, preferably for 1 hour.
Embodiment 28. The method of any one of Embodiments 26 and 27, wherein subsequent to the incubation, the fluid containing activated carbon and the dicarboxylic acid is filtered, whereby upon filtration a decolorized fluid is obtained containing the dicarboxylic acid.
Embodiment 29. The method of Embodiment 28, wherein the decolorized fluid containing the dicarboxylic acid is subjected to a crystallization step, wherein the dicarboxylic acid is crystallized from the decolorized, the dicarboxylic acid containing fluid in the crystallization step providing crystallized dicarboxylic acid and a supernatant.
Embodiment 30. The method of Embodiment 29, wherein the crystallization is performed at a temperature of 28 °C or less, preferably the crystallization is performed at a temperature of between about 10°C and about 28°C.
Embodiment 31. The method of any one of Embodiments 29 to 30, wherein the crystallized dicarboxylic acid is removed from the supernatant, preferably by centrifugation or filtration.
Embodiment 32. The method of Embodiment 31, wherein centrifugation is effected by means of a pusher centrifuge.
Embodiment 33. The method of Embodiment 31, wherein filtration is effected by means of a drum filter.
Embodiment 34. The method of any one of Embodiments 31 to 33, wherein the crystallized dicarboxylic acid removed from the supernatant is subjected to a washing step and/or drying step.
Embodiment 35. The method of Embodiment 34, wherein the drying step is effected by means of a fluidized bed dryer, preferably the fluidized bed dryer is operated at standard pressure and impinged with hot air.
Embodiment 36. The method of any one of Embodiments 1 to 35, wherein the dicarboxylic acid is a dicarboxylic acid of 10 carbon atoms, 12 carbon atoms, 14 carbon atoms, or 16 carbon atoms.
Embodiment 37. The method of Embodiment 36, wherein the dicarboxylic acid is a saturated dicarboxylic acid.
Embodiment 38. The method of any one of Embodiments 36 to 37, wherein the dicarboxylic acid is a linear dicarboxylic acid.
Embodiment 39. The method of any one of Embodiments 36 to 38, wherein the dicarboxylic acid is a linear saturated carboxylic acid.
Embodiment 40. The method of any one of Embodiments 36 to 39, wherein the dicarboxylic acid is selected from the group comprising decanedioic acid, dodecanedioic acid, tetradecanedioic acid and hexadecanedioic acid, preferably the dicarboxylic acid is dodecanedioic acid (DDDA).
Embodiment 41. The method of any one of Embodiments 1 to 40, wherein the precursor compound comprises an ethyl ester or methyl ester of the monocarboxylic acid the dicarboxylic acid to be produced.
Embodiment 42. The method of Embodiment 41, wherein the precursor is or comprises
   an ethyl ester or methyl ester, preferably an ethyl ester, of decanoic acid or sebacid acid in case the dicarboxylic acid to be produced is decanedioic acid;
   an ethyl ester or methyl ester, preferably an ethyl ester, of dodecanoic acid or lauric acid in case the dicarboxylic acid to be produced is dodecanedioic acid;
   an ethyl ester or methyl ester, preferably an ethyl ester, of tetradecanoic acid in case the dicarboxylic acid to be produced is tetradecanedioic acid; and
   an ethyl ester or methyl ester, preferably an ethyl ester, of hexadecanoic acid in case the dicarboxylic acid to be produced is hexadecanedioic acid.
Embodiment 43. The method of any one of Embodiments 1 to 40, wherein the precursor compound comprises an alkane compound.
Embodiment 44. The method of Embodiment 43, wherein the precursor compound is selected from the group comprising decane, dodecane, tetradecane and hexadecane.
Embodiment 45. The method of any one of Embodiments 43 to 44, wherein the precursor compound is or comprises
   decane in case the dicarboxylic acid to be produced is decanedioic acid;
   dodecane in case the dicarboxylic acid to be produced is dodecanedioic acid;
   tetradecane in case the dicarboxylic acid to be produced is tetradecanedioic acid; and
   hexadecane in case the dicarboxylic acid to be produced is hexadecanedioic acid.
Embodiment 46. The method of any one of Embodiments to 45, wherein in the bioconversion step bioconversion of the precursor compound is effected by a biocatalyst.
Embodiment 47. The method of Embodiment 46, wherein the biocatalyst is a microorganism.
Embodiment 48. The method of claim 47, wherein the microorganism is a yeast.
Embodiment 49. The method of any one of Embodiments 47 and 48, wherein the yeast expresses the omega-oxidation pathway, preferably expressed the omega-oxidation pathway upon induction.
Embodiment 50. The method of any one of Embodiments 47 to 49, wherein the microorganism is not capable of beta-oxidation.
Embodiment 51. The method of any one of Embodiments 47 to 50, wherein the microorganism is produced by means of fermentation in a fermentation broth.
Embodiment 52. The method of Embodiment 51, wherein at the end of the fermentation, the temperature of the fermentation broth is increased from a temperature, preferably a fermentation temperature, of about 20 °C to about 28 °C to an inactivation temperature of about 60°C to about 90 °C.
Embodiment 53. The method of Embodiment 52, wherein the fermentation broth is kept at the inactivation temperature for about 30 minutes to about 90 minutes, preferably for about 60 minutes.
Embodiment 54. The method of any one of Embodiments 51 to 53, wherein at the end of the fermentation the pH of the fermentation broth is increased to a pH of about 8 to 11, preferably to a pH of about 8 to about 9, more preferably to a pH of about 8.5.
Embodiment 55. The method of Embodiment 54, wherein the pH increased by adding ammonia to the fermentation broth, preferably the ammonia is ammonia solution of up to 32 % (wt/wt), preferably the ammonia solution is from about 10 % (wt/wt) to about 32 % (wt/wt), more preferably the ammonia solution is about 25 % (wt/wt).
Embodiment 56. The method of any one of Embodiments 1 to 55, wherein the fermentation broth is separated from the cell, preferably by means of centrifugation.
Embodiment 57. The method of Embodiment 56, wherein a microorganism concentrate and a clarified broth is obtained.
Embodiment 58. The method of Embodiment 57, wherein the clarified broth is subject to a further step, wherein in the further step biomass and/or cell debris is removed from the clarified broth and a further clarified broth is obtained.
Embodiment 59. The method of Embodiment 58, wherein removal of the biomass and/or cell debris are removed from the clarified broth by means of ultrafiltration.
Embodiment 60. The method of any one of Embodiments 57 to 59, wherein the clarified broth and/or the further clarified broth are the medium containing the dicarboxylic acid which is subjected to the purification step.
Embodiment 61. A method for the production of a dicarboxylic acid, wherein the method comprises
   - a bioconversion step, wherein in the bioconversion step, the dicarboxylic acid is produced from a precursor compound contained in a medium; and a
   - purification step, wherein the purification step comprises a distillation step, wherein in the distillation step, the dicarboxylic acid obtained from the bioconversion step acid is subjected to distillation.
Embodiment 62. The method of Embodiment 61, wherein the dicarboxylic acid obtained from the bioconversion step or from a stage of the purification step carried out prior to the distillation step is obtained as precipitated dicarboxylic acid, preferably the dicarboxylic acid is obtained as precipitated dicarboxylic acid from an acidification step, and wherein the precipitated dicarboxylic acid is melted, preferably at a temperature of about 140 °C, and the melted dicarboxylic acid subjected to distillation.
Embodiment 63. The method of any one of Embodiments 61 to 62, wherein in the distillation step, the melted dicarboxylic acid is heated so as to obtain vaporized dicarboxylic acid, preferably the melted dicarboxylic acid is heated in a distillation column so as to obtain vaporized dicarboxylic acid.
Embodiment 64. The method of Embodiment 63, wherein in the distillation column vaporized dicarboxylic acid is separated from a high-boiler and/or a low-boiler, preferably a high-boiler and/or a low-boiler comprised in or associated with the precipitated dicarboxylic acid.
Embodiment 65. The method of any one of Embodiments 63 to 64, wherein the dicarboxylic acid is vaporized at a temperature of about 190 °C to about 240°C.
Embodiment 66. The method of any one of Embodiments 63 to 65, wherein the dicarboxylic acid is vaporized at a pressure of 1 hPa to 10 hPa.
Embodiment 67. The method of any one of Embodiments 65 to 66, wherein the conditions for vaporization of the dicarboxylic acid ranges from 190°C at 1 hPa to about 240°C at 10 hPa.
Embodiment 68. The method of any one of Embodiments 63 to 67, wherein the dicarboxylic acid is vaporized in a thin-film evaporator, wherein in the thin-film evaporator the high-boiler is separated from the dicarboxylic acid.
Embodiment 69. The method of Embodiment 68, wherein the dicarboxylic acid obtained from the thin-film evaporator is conducted to a rectification column, wherein in the rectification column the dicarboxylic acid is separated from the low-boiler.
Embodiment 70. The method of Embodiment 69, wherein the dicarboxylic acid is introduced to a feed tray at the middle section of the rectification column.
Embodiment 71. The method of any one of Embodiments 69 to 70, wherein the rectification column comprises at least eight trays.
Embodiment 72. The method of any one of Embodiments 69 to 71, wherein the dicarboxylic acid is introduced into the rectification column at a temperature of about 190 °C to about 240 °C.
Embodiment 73. The method of any one of Embodiments 69 to 72, wherein the dicarboxylic acid is introduced into the rectification column at a pressure of about 1 hPA to about 10 hPa.
Embodiment 74. The method of any one of Embodiments 69 to 73, wherein the dicarboxylic acid is introduced into the rectification column at temperature/pressure ranges from 190°C at 1 hPa to about 240°C at 10 hPa.
Embodiment 75. The method of any one of Embodiments 69 to 74, wherein the dicarboxylic acid obtained in the distillation step is removed from the bottom of the rectification column.
Embodiment 76. The method of any one of Embodiments 61 to 75, wherein the purification step comprises a nano-diafiltration step, wherein the nanofiltration step is carried out prior to the distillation step.
Embodiment 77. The method of Embodiment 76, wherein the nanofiltration step is a stage of the purification step, preferably a stage of the purification step carried out prior to the distillation step.
Embodiment 78. The method of any one of Embodiments 76 to 77, wherein the dicarboxylic acid obtained from the bioconversion step is subjected to the nano-diafiltration step and the dicarboxylic acid obtained from the nano-diafiltration step subjected to the distillation step.
Embodiment 79. The method of any one of Embodiments 76 to 78, wherein the nanofiltration step provides the dicarboxylic acid as precipitated dicarboxylic acid.
Embodiment 80. The method of any one of Embodiments 76 to 79, wherein the medium obtained from the bioconversion step containing the dicarboxylic acid is subjected to the nano-diafiltration step, wherein the retentate of the nano-diafiltration contains the dicarboxylic acid.
Embodiment 81. The method of any one of Embodiments 76 to 80, wherein the membrane used in the nano-diafiltration step has a cut-off value of between 150 Da and 300 Da, preferably the cut-off value is ≤ 150 Da.
Embodiment 82. The method of any one of Embodiments 80 to 81, wherein the dicarboxylic acid containing retentate of the nano-diafiltration step is subjected to an acidification step.
Embodiment 83. The method of Embodiment 82, wherein in the acidification step, sulfuric acid is added to the dicarboxylic acid containing retentate of the nano-diafiltration step and the dicarboxylic acid is precipitated from the retentate, and the precipitated dicarboxylic acid is optionally washed.
Embodiment 84. The method of any one of Embodiments 61 to 83, wherein the dicarboxylic acid is a dicarboxylic acid of 10 carbon atoms, 12 carbon atoms, 14 carbon atoms, or 16 carbon atoms.
Embodiment 85. The method of Embodiment 84, wherein the dicarboxylic acid is a saturated dicarboxylic acid.
Embodiment 86. The method of any one of Embodiments 84 to 85, wherein the dicarboxylic acid is a linear dicarboxylic acid.
Embodiment 87. The method of any one of Embodiments 84 to 86, wherein the dicarboxylic acid is a linear saturated carboxylic acid.
Embodiment 88. The method of any one of Embodiment 84 to 87, wherein the dicarboxylic acid is selected from the group comprising decanedioic acid, dodecanedioic acid, tetradecanedioic acid and hexadecanedioic acid, preferably the carboxylic acid is dodecanedioic acid (DDDA).
Embodiment 89. The method of any one of Embodiments 61 to 88, wherein the precursor compound comprises an ethyl ester or methyl ester of the monocarboxylic acid of the dicarboxylic acid to be produced.
Embodiment 90. The method of Embodiment 89, wherein the precursor is or comprises
   an ethyl ester or methyl ester, preferably an ethyl ester, of decanoic acid or sebacid acid in case the dicarboxylic acid to be produced is decanedioic acid;
   an ethyl ester or methyl ester, preferably an ethyl ester, of dodecanoic acid or lauric acid in case the dicarboxylic acid to be produced is dodecanedioic acid;
   an ethyl ester or methyl ester, preferably an ethyl ester, of tetradecanoic acid in case the dicarboxylic acid to be produced is tetradecanedioic acid; and
   an ethyl ester or methyl ester, preferably an ethyl ester, of hexadecanoic acid in case the dicarboxylic acid to be produced is hexadecanedioic acid.
Embodiment 91. The method of any one of Embodiments 1 to 88, wherein the precursor compound comprises an alkane compound.
Embodiment 92. The method of Embodiment 91, wherein the precursor compound is selected from the group comprising decane, dodecane, tetradecane and hexadecane.
Embodiment 93. The method of any one of Embodiment 91 to 92, wherein the precursor compound is or comprises
   decane in case the dicarboxylic acid to be produced is decanedioic acid;
   dodecane in case the dicarboxylic acid to be produced is dodecanedioic acid;
   tetradecane in case the dicarboxylic acid to be produced is tetradecanedioic acid; and
   hexadecane in case the dicarboxylic acid to be produced is hexadecanedioic acid.
Embodiment 94. The method of any one of Embodiments 61 to 93, wherein in the bioconversion step bioconversion of the precursor compound is effected by a biocatalyst.
Embodiment 95. The method of Embodiment 94, wherein the biocatalyst is a microorganism.
Embodiment 96. The method of Embodiment 95, wherein the microorganism is a yeast.
Embodiment 97. The method of any one of Embodiments 95 and 96, wherein the yeast expresses the omega-oxidation pathway, preferably expressed the omega-oxidation pathway upon induction.
Embodiment 98. The method of any one of Embodiments 95 to 97, wherein the microorganism is not capable of beta-oxidation.
Embodiment 99. The method of any one of Embodiments 95 to 98, wherein the microorganism is produced by means of fermentation in a fermentation broth.
Embodiment 100. The method of Embodiment 99, wherein at the end of the fermentation, the temperature of the fermentation broth is increased from a temperature, preferably a fermentation temperature, of about 20 °C to about 28 °C to an inactivation temperature of about 60°C to about 90 °C.
Embodiment 101. The method of Embodiment 100, wherein the fermentation broth is kept at the inactivation temperature for about 30 minutes to about 90 minutes, preferably for about 60 minutes.
Embodiment 102. The method of any one of Embodiments 99 to 101, wherein at the end of the fermentation the pH of the fermentation broth is increased to a pH of about 8 to 11, preferably to a pH of about 8 to about 9, more preferably to a pH of about 8.5.
Embodiment 103. The method of Embodiment 102, wherein the pH increased by adding ammonia to the fermentation broth, preferably the ammonia is ammonia solution of up to 32 % (wt/wt), preferably the ammonia solution is from about 10 % (wt/wt) to about 32 % (wt/wt), more preferably the ammonia solution is about 25 % (wt/wt).
Embodiment 104. The method of any one of Embodiments 61 to 103, wherein the fermentation broth is separated from the cell, preferably by means of centrifugation.
Embodiment 105. The method of Embodiment 104, wherein a microorganism concentrate and a clarified broth is obtained.
Embodiment 106. The method of Embodiment 105, wherein the clarified broth is subject to a further step, wherein in the further step biomass and/or cell debris is removed from the clarified broth and a further clarified broth is obtained.
Embodiment 107. The method of Embodiment 106, wherein removal of the biomass and/or cell debris are removed from the clarified broth by means of ultrafiltration.
Embodiment 108. The method of any one of Embodiments 105 to 107, wherein the clarified broth and/or the further clarified broth are the medium containing the dicarboxylic acid which is subjected to the purification step.
Embodiment 109. A dicarboxylic acid obtainable by a method of any one of Embodiments 1 to 108.
Embodiment 110. Use of a nano-diafiltration device in a method of producing and/or purifying a dicarboxylic acid.
Embodiment 111. Use of Embodiment 110, wherein the nanodiafiltration device is one as described in any one of Embodiments 1 to 108.
Embodiment 112. Use of nano-diafiltration in a method of producing and/or purifying a dicarboxylic acid.
Embodiment 113. Use of Embodiment 112, wherein the nano-diafiltration is one as described in any one of Embodiments 1 to 108.
Embodiment 114. Use of any one of Embodiments 110 to 113, wherein the method is a method of any one of embodiments 1 to 108.
Embodiment 115. Use of a distillation step in a method of producing and/or purifying a dicarboxylic acid.
Embodiment 116. Use of Embodiment 115, wherein distillation step is one as described in any one of Embodiments 1 to 108.
Embodiment 117. Use of a thin-film evaporator in a method of producing and/or purifying a dicarboxylic acid.
Embodiment 118. Use of Embodiment 117, wherein the thin-film evaporator as described in any one of Embodiments 1 to 108.
Embodiment 119. Use of any one of Embodiments 117 to 118, wherein a rectification column is attached to the thin-film evaporator, wherein preferably vaporized dicarboxylic acid is conducted from the thin-film evaporator to the rectification column.
Embodiment 120. Use of any one of Embodiments 115 to 119, wherein the method is a method of any one of Embodiments 1 to 108.
Embodiment 121. Use of any one of Embodiments 110 to 120, wherein the dicarboxylic acid is a dicarboxylic acid of 10 carbon atoms, 12 carbon atoms, 14 carbon atoms, or 16 carbon atoms.
Embodiment 122. The method of Embodiment 121, wherein the dicarboxylic acid is a saturated dicarboxylic acid.
Embodiment 123. The method of any one of Embodiments 121 to 122, wherein the dicarboxylic acid is a linear dicarboxylic acid.
Embodiment 124. The method of any one of Embodiments 121 to 123, wherein the dicarboxylic acid is a linear saturated carboxylic acid.
Embodiment 125. The method of any one of Embodiments 121 to 124, wherein the dicarboxylic acid is selected from the group comprising decanedioic acid, dodecanedioic acid, tetradecanedioic acid and hexadecanedioic acid, preferably the carboxylic acid is dodecanedioic acid (DDDA).

The present inventors have surprisingly found that the method for the production of a dicarboxylic of the present invention comprising, preferably as a purification step, a nano-diafiltration step and/or a distillation step, preferably comprising a thin-film evaporator, is providing a dicarboxylic acid which is essentially free of impurities such as salts, organic sugars, amino acids and derivatives thereof. Also, the present inventors have surprisingly found that the use of nano-diafiltration and distillation according to the present invention, either alone or in combination, allows the purification of a dicarboxylic acid essentially free of said impurities from a product obtained from a bioconversion step containing the dicarboxylic acid, preferably a bioconversion step as disclosed herein. The absence of such impurities results in less undesired follow-up reactions when using the accordingly produced dicarboxylic acid and dodecanedioic acid in particular, for example in polymerization. In other words, the dicarboxylic acid provided in accordance with the present invention shows purity comparable, at least in terms of avoiding any undesired follows-up reactions in polymerization, to chemically synthesized dicarboxylic acids.

The present inventors have surprisingly found that the use of a nano-diafiltration step in a method for producing dicarboxylic acid provides for an advantageous overall process for the production of dicarboxylic acid. More specifically, the advantage arises from purification of dicarboxylic acid obtained after separation of any biomass such as cells and cell debris from the medium in which the bioconversion of dicarboxylic acid is performed. In an embodiment, purity of the dicarboxylic acid, preferable of DDDA, is about 95 %, more preferably of ≥ 98.5. Typically, such medium is a fermentation broth used in the cultivation of a microorganism and a yeast in particular, whereby the microorganism performs the bioconversion of a precursor compound such as ethyl laurate contained in the medium, preferably contained in the fermentation broth.

Similarly, the present inventors have surprisingly found that the use of a distillation step in a method for producing dicarboxylic acid provides for an advantageous overall process for the production of dicarboxylic acid. More specifically, the advantage arises from purification of dicarboxylic acid obtained after separation of any biomass such as cells and cell debris from the medium in which the bioconversion of dicarboxylic acid is performed. In an embodiment, purity of the dicarboxylic acid, preferable of DDDA, is about 95 %, more preferably of ≥ 98.5. Typically, such medium is a fermentation broth used in the cultivation of a microorganism and a yeast in particular, whereby the microorganism performs the bioconversion of a precursor compound such as ethyl laurate contained in the medium, preferably contained in the fermentation broth.

Additionally, the present inventors have found that adding of ammonia after the bioconversion of the precursor compound into a dicarboxylic acid, typically at the end of the fermentation step, so as to increase the pH from about 5.8 as used in the bioconversion step providing the biocatalyst, preferable the microorganism, carrying out the bioconversion, to a pH of about 8 to 9, is advantageous particularly if after the nano-filtration step the dicarboxylic acid containing retentate of the nano-filtration step is subjected to an acidification step, wherein the acidification makes use of sulfuric acid. It is also within the present invention, particularly the second aspect of the present invention, that such increase in pH to about 8 to 9 is advantageous if prior to the distillation step, the medium from the bioconversion step, preferably after removing the biomass of the biocatalyst performing the bioconversion, and optionally after subsequent ultrafiltration, is subjected to an acidification step, wherein the acidification makes use of sulfuric acid.

Finally, the present inventors have surprisingly found that the use of a thin-film evaporator in the production and/or purification of a dicarboxylic acid in accordance with the present invention, does not result in the formation of an anhydride and/or other undesired reaction products of the dicarboxylic acid.

In an embodiment, sulfuric acid is from about 78 % (wt/wt) to about 98 % (wt/wt), preferably about 98 % (wt/wt). Under such conditions, not only a very pure dicarboxylic acid devoid of side-products such as lauric acid and/or hydroxylauric acid, but also a very pure ammonium sulphate is obtained. The purity obtained for the dicarboxylic acid is typically ≥ 98.5 %, more typically about 99.5 %, and the purity of the ammonium sulphate is typically ≥ 95 %, more typically about 99.5 %. Accordingly, the method according to the present invention allows the production of a dicarboxylic acid while decreasing any further resources consuming polisihing methods. Also, very pure ammonium sulphate is obtained as a side-product which may be directly used as a fertilizer or in the fertilizer industry. This is advantageous over processes over the prior art using sodium hydroxide solution which results in high sodium sulfate levels in waste water.

It is within the present invention that the method for the production of a dicarboxylic acid comprises a purification step. Such purification step comprises a nano-diafiltration step and/or distillation step, whereby the dicarboxylic acid is purified by both the nano-diafiltration step and the distillation step. The method for the production of a dicarboxylic acid according to the invention may comprise a nano-diafiltration step as disclosed herein, but no distillation step as disclosed herein. Alternatively, the method for the production of a dicarboxylic acid according to the invention may comprise a distillation step as disclosed herein, but no nano-diafiltration step as disclosed herein. It will be appreciated by a person skilled in the art that the overall method has to be adapted depending on whether or not such nano-diafiltration step or such distillation step is to be carried out or not. Such adaptation applies in particular to the acidification step. In case of a method of the invention making use of a nano-diafiltration step, the retentate of the nano-diafiltration step is subjected to such acidification step; in case of a method of the invention making use of a distillation step and not making use of a nano-diafiltration step, the medium obtained from the bioconversion step is subjected to the distillation step, preferably after removal of the biomass of the biocatalyst and after subsequent subjecting of the biomass-free medium to an ultrafiltration step, preferably an ultrafiltration step as described herein; in an embodiment, the ultrafiltered medium is subjected to an acidification step resulting in the formation of a precipitate which is ultimately subject to the distillation step.

In a preferred embodiment of the invention, the dicarboxylic acid is dodecanedioic acid (DDDA).

In an embodiment of the invention, DDDA prepared in accordance therewith meets the current industry specification as summarized in Table 1.

**Table 1: Industry specification for DDDA**

| **Characteristic** | **Measurement** | **Requirement** |
|---|---|---|
| *Appearance* | visual | Granules, flakes or beads (not powder) |
| *Purity* | Mass % | 98.6 min |
| *Acid No* | mg KOH/gm. | 480-495 |
| *Moisture* | % | 0.4 max |
| *Color* | APHA | 15 max |
| *Ash* | ppm | 2 max |
| *Iron* | ppm | 1 max |
| *Nitrogen* | ppm | 34 max |
| *Monobasic Acids** | Mass % | 0.08 max |
| *Other Dibasic Acids* | Mass % | 1.0 max |

According to the present invention, the method for producing the dicarboxylic acid by bioconversion from a precursor compound comprises a purification step, wherein the purification step comprises a nano-diafiltration step. This nano-diafiltration step is particularly effective in purifying a medium comprising dicarboxylic acid such as a fermentation broth, and in concentrating and isolating, respectively, the dicarboxylic acid.

The dicarboxylic acid containing medium, preferably obtained from an ultrafiltration step of a dicarboxylic acid such as DDDA containing medium, such as a fermentation broth, is subjected to a nano-diafiltration device such a membrane module having a cut-off value between 150 Da and 300 Da, preferably a cut-off value of ≤ 150 Da. In an embodiment of the invention where the dicarboxylic acid containing medium comprises ammonia salt, the diammonia salt of the dicarboxylic acid remains at the retentate side of the nano-diafiltration device und is concentrated. Such concentration may result in partial precipitation of the ammonia salt of the dicarboxylic acid such as DDDA if the solubility limit of said dicarboxylic acid is exceeded. The permeate of the nano-diafiltration device contains C1 to C6 compounds such as succinic acid or formic acid, monosaccharides such as glucose, amino acids, salts and trace elements. The removal of succinic acid from the product stream is particularly advantageous as succinic acid interferes with polymerization using the dicarboxylic acid, particularly the polymerization of dodecanedioic acid into polyamide and polyester.

The separation of these compounds contained in the permeate from the dicarboxylic acid and its salts is advantageous over the methods of the prior art for producing a dicarboxylic acid which use activated carbon or two crystallization steps. Because of said separation the salts and more specifically the diammonium salt of the dicarboxylic acid contained in the retentate may be split by acid such as sulfuric acid into the dicarboxylic acid and ammonium sulphate both of which are chemically pure.

Suitable membranes for use in nano-diafiltration and a nano-diafiltration device, respectively, are known to a person skilled in the art and commercially available (for example from GE Osmonics (Minnetonka, MI; USA). In an embodiment of the invention, a suitable membrane has a molecular weight cut-off (MWCO) of 150 to 300 Da. Because of this MWCO, the membrane retains molecules having a molecular weight of about 150 Da to about 300 Da. Furthermore, this kind of membrane retains bivalent and multivalent ions, whereas monovalent ions transit into the permeate. Since the monovalent ions pass through the membrane, they do not contribute to any osmotic pressure which allows operating these membranes at high pressure; in an embodiment the pressure is up to 40 bar. In an embodiment, the membrane is present as a spiral wound flat sheet; in an alternative embodiment, the membrane is present as a hollow fibre.

The membranes used in a nano-diafiltration and a nano-diafiltration device, respectively, are, in an embodiment, made of one or the following materials: polyacrylenitrile (PAN), polyethersulphone (PES) and polyvinylidene fluoride (PVDF).

The acidification step is, due to the use of strong acids such as sulfuric acid, typically carried out in a corrosion-resistant agitated reactor. Preferably, the reactor is ceramic lined or the material of construction is Hastelloy or any other acid resistant material. In an embodiment, the sulfuric acid used in the acidification step is about 98 % (wt/wt).

In an embodiment of the method for producing the dicarboxylic acid according to the first and the second aspect, the acidification step comprises one, several or all of the following steps. In a first step, the nano-diafiltration retentate is mixed with activated carbon to capture color forming compounds, and then filtered to remove the carbon. In a second step, the thus obtained solution is acidified to precipitate out the dicarboxylic acid and then separated from the mother liquor. The acidification process is preferably completed in a batch mode. In an alternative embodiment, the acidification step can be performed as continuous acidification or in a loop reactor.

In an embodiment of the method for producing the dicarboxylic acid according to the first and the second aspect, the dicarboxylic acid precipitated in the acidification step is subjected to a melting step, wherein in the melting step the precipitated dicarboxylic acid is melted. In an embodiment, the precipitated dicarboxylic acid is dried in a fluidized bed dryer and fed, preferably with a screw conveyor, to a melting device where the dicarboxylic acid is melted. In a further embodiment, the thus melted dicarboxylic acid is conducted by means of a screw conveyer to the thin-film evaporator.

For the thermal separation of a mixture in a thin-film evaporator a thin film is produced at the heated wall of a cylindrical or conical evaporator. A distribution ring on the rotor distributes the liquid evenly across the periphery. Then, the blades fitted at the rotor spread the liquid as a thin film of min. 0.5 mm over the heat transfer surface.

The model concept for the flow in the thin film evaporator assumes that prior to each rotor blade a bow wave is formed. In the gap between the rotor blade and the heating surface, fluid is supplied from the bow wave of a highly turbulent area with intense heat and mass transport. This results in a good heat transfer performance even with viscous products. In addition, the formation of deposits is avoided and the intensive mixing also protects temperature-sensitive products from overheating.

Another important task of the rotor is to stabilize the liquid film on the heating surface at high evaporation rates. On the one hand evaporation in the area of nucleate boiling is possible without ruptures of the film. On the other hand, the liquid film is pressed against the heating surface by the centrifugal force. This avoids the adverse evaporation mode, in which a vapour layer with insulating effect is formed under the liquid film. Therefore, due to the functional principle extremely high specific evaporation rates are achievable in thin film evaporators.

In an embodiment of the invention, the thin-film evaporator comprises a rectification column, preferably the rectification column replaces a condenser typically comprised by a thin-film evaporator of the art.

In an embodiment of the method according to the second and the first aspect, melted dicarboxylic acid is subject to a distillation step. Preferably precipitated dicarboxylic acid, optionally washed after precipitation, is dried. Preferably, such drying is carried out in a fluidized bed dryer. In an embodiment, the drying of dicarboxylic acid is performed at a temperature of about 108 °C to about 132 °C, preferably at a temperature of about 120°C. Preferably, the drying of dicarboxylic acid is performed at atmospheric pressure. The dried dicarboxylic acid is preferable molten at 140 °C and subjected to thin film evaporation.

In an embodiment of the method according to the second and the first aspect, molten, i.e. liquid DDDA is directed to an evaporator. In a preferred embodiment, the evaporator is a thin-film evaporator. The evaporator separates one or more high-boilers from the molten dicarboxylic acid which is also referred to as raw dicarboxylic acid. Such raw dicarboxylic acid may comprise, among others, proteins, amino acids, polysaccharides, long-chain non-oxidized fatty acids and lactams.

In an embodiment and as preferably used herein, a high-boiler is a chemical compound the boiling point of which is higher than the boiling point of the dicarboxylic acid. A high-boiler may be or may comprise a protein, an amino acid, a polysaccharide and/or a caramelized carbohydrate.

In an embodiment of the method according to the second and first aspect, the raw dicarboxylic acid depleted of (a) high-boiler(s) is transferred into a rectification column. In said rectification column, one or more low-boilers are removed and distilled dicarboxylic acid obtained.

In an embodiment and as preferably used herein, a low-boiler is a chemical compound the boiling point of which is lower than the boiling point of the dicarboxylic acid. A low-boiler may be or may comprise an amino acid, short-chain dicarboxylic acids, lactams and/or non-oxidized fatty acids.

In an embodiment of the invention, the dicarboxylic acid obtained from the acidification step and/or a subsequent filtering and/or washing step is subjected to a crystallization rather than distillation.

In this embodiment, the dicarboxylic acid is dissolved in a fluid and, optionally, activated carbon is added. The dicarboxylic acid and, respectively, the mixture of dissolved dicarboxylic acid and activated carbon is preferably kept at an increased temperature, preferably a temperature at which the dicarboxylic acid was dissolved. In a preferred embodiment, dicarboxylic acid and the mixture containing dicarboxylic acid and the activated carbon are maintained at about 90°C or higher for about 30 minutes to about 2 hours. In another embodiment, dicarboxylic acid and the mixture containing dicarboxylic acid and the activated carbon are kept at 90°C for 1 hour. Subsequently, the dicarboxylic acid or the mixture is filtered to produce a decolorized solution that includes dicarboxylic acid. The decolorized solution is preferably cooled to about 28°C or lower. By decreasing the temperature, dicarboxylic acid crystalizes from the fluid.

In an embodiment, the fluid is water, an organic solvent or a mixture of water and an organic solvent. Preferably, the organic solvent is acetic acid.

In an embodiment, the dicarboxylic acid crystals are separated from the decolorized fluid, preferably by centrifugation or filtration.

In an embodiment, crystals of the dicarboxylic acid are washed and dried. Preferably, the crystals are washed with water at about 20°C, more preferably such washing is performed in the centrifugation step, preferably in a pusher centrifuge .

In an embodiment of the invention method for producing a dicarboxylic acid according to the first and the second aspect, the method comprises an ultrafiltration step. Such ultrafiltration step removes the biomass and/or cell debris from the medium used in the bioconversion step. Preferably, such medium is a fermentation broth which has been subject to one or more of the following steps: cell separation and ammonia addition.

In the ultrafiltration step and/or in an ultrafiltration device used in the ultrafiltration step, a polymer membrane or a ceramic module may be used. The membrane and the module, respectively, can be designed as hollow fiber or Spiral Wound Flat Sheet. In an embodiment, the molecular weight cut off (MWCO) of the membrane and of the ceramic module is between 5.000 Da and 40.000 Da, preferably the MWCO of the membrane and of the ceramic module is 20.000 Da. In an embodiment, the material of the membrane is polysulphone or polyethersulphone.

In an embodiment, the ultrafiltration is carried out at a temperature of about 40 °C in standard operation. In an alternative embodiment, the ultrafiltration is performed as diafiltration. It will be appreciated by a person skilled in the art that if the ultrafiltration is run as diafiltration, the losses of the dicarboxylic acid are less.

The permeate from the ultrafiltration step is free of biomass such as cells and cell particles as well as higher molecular peptides. The retentate of the ultrafiltration is preferably subjected to waste water treatment, whereas the permeate is subjected to the nano-diafiltration step.

In an embodiment of the method for producing a dicarboxylic acid according to the first and the second aspect, the method comprises a cell separation step.

In an embodiment, said cell separation step comprises centrifugation step, wherein in the centrifugation step biomass and/or cell debris from the medium used in the bioconversion step. Preferably, such medium is a fermentation broth which has been subjected to ammonia addition.

In an embodiment, in the separation step, the medium, preferably the fermentation broth and more preferably a fermentation broth to which ammonia has been added, has a temperature of 60°C and a pH of about 8 to about 9, preferably about 8.5. Preferably, a centrifuge is utilized to clarify the medium. This step produces clarified broth (centrate) separated from the biomass, such as a microorganism and preferably a yeast, carrying out the bioconversion step.

In an embodiment, standard centrifuge technology capable of yeast cell removal is acceptable. Preferably, the centrifuge technology is disc stack or nozzle centrifuge technology. In a preferred embodiment, the separation step produces a centrate to concentrate (Volume Concentration Factor VCF) volume ratio of 1.32.

In an embodiment of the method for producing a dicarboxylic acid according to the first and the second aspect, the method comprises a solvation step. In such solvation step, the pH of the medium, preferably the fermentation broth, is increased. Preferably, the solvation step is performed at the end of the fermentation step and after killing of the microorganism if the bioconversion step is carried out by a microorganism. Such killing is preferably carried out by increasing the temperature of the medium to about 60°C, whereby, preferably, the temperature is held for about one hour. More specifically, in such solvation step the pH of the medium is increased to between 8 and 9, preferably to 8.5 with ammonia. Preferably, ammonia is provided as solution in water, wherein the ammonia concentration is up to 32%, preferably about 25 %. In such solvation step, DDDA is dissolved.

In an embodiment, the heating and the solvation step are carried out subsequently and separately.

In an embodiment, the heating and the solvation step are carried out simultaneously or at such that they at least overlap over a certain period of time.

In a further embodiment, the heating and the solvation step are performed in a vessel different from the vessel where the bioconversion step is carried out.

In an embodiment of the method for producing a dicarboxylic acid according to the first and the second aspect, the precursor compound is an ethyl ester or a methyl ester of a monocarboxylic acid of the dicarboxylic acid to be produced. In a preferred embodiment, the dicarboxylic acid to be produced is dodecanedioic acid and the precursor compound is ethyl laurate.

In an embodiment, ethyl laurate is commercially available ethyl laurate. In an alternative embodiment, ethyl laurate is prepared by ethyl esterification of lauric acid.

In an embodiment, ethyl laurate used as precursor compound in the method according to the first and the second aspect contains 89 - 92% ethyl laurate (wt/wt)); ethyl esters of C10 and C14 fatty acids may make up about 1% (wt/wt) each of the precursor compound.

In an alternative embodiment of the method for producing a dicarboxylic acid according to the first and the second aspect, the precursor compound is an alkane compound the terminal carbon atoms of which are oxidized, preferably in the bioconversion step, so as to form a carboxylic acid group at each end. For example, if the dicarboxylic acid to be produced by the methods of the invention, preferable the method of the invention according to the first and the second aspect, is dodecanedioic acid, a preferred precursor compound is dodecane.

In an embodiment of the method for producing a dicarboxylic acid according to the first and the second aspect, the precursor compound is converted into the dicarboxylic acid by means of a microorganism. In a preferred embodiment, the microorganism a yeast, preferably the yeast is Candida viswanathii, more preferably a genetically engineered Candida viswanathii. In a preferred embodiment the yeast is one as disclosed in US 9,909,152.

In an embodiment the yeast expresses the omega-oxidation pathway, preferably expressed the omega-oxidation pathway upon induction.

In an embodiment, the microorganism, preferably the yeast and more preferably *Candida viswanathii* is not capable of beta-oxidation.

In an embodiment of the method for producing a dicarboxylic acid according to the first and the second aspect, the method comprises a fermentation step. In such fermentation step, a microorganism is prepared and put in condition to prepare or carry out the bioconversion step.

The dicarboxylic acid, preferably DDDA, is produced through bioconversion, whereby such bioconversion is preferably performed by a genetically-engineered strain of Candida viswanathii which is produced by a fermentation step. In an embodiment, the fermentation is performed as fed batch process comprising a rapid growth phase. The two feed streams are dextrose and the precursor compound such as ethyl laurate, whereby ethyl laurate is the precursor compound for the dicarboxylic acid which may be added concomitantly with dextrose or subsequent to dextrose addition. The yeast strain takes up the precursor compound such as ethyl laurate, cleaves off the ethanol, and converts the fatty acid into the dicarboxylic acid. The resulting ethanol can be metabolized by the strain. Alternatively, the yeast strain takes up an alkane such as dodecane, and converts the alkane into the dicarboxylic acid. In an embodiment, the length of the precursor compound in terms of the number of C atoms is the same as the length of the dicarboxylic acid to be produced, preferably by means of bioconversion.

In an embodiment, the growth phase, which includes a seed strain, is conducted with dextrose as the carbon source and fermentation conditions that foster rapid biomass accumulation. Cells are grown aerobically to high cell density on minimal media in seed bioreactors and then transferred to a production fermenter. Growth phase is continued in the production fermenter until dextrose is exhausted. The medium provides a nitrogen level that will leave an excess of nitrogen at the end of the growth phase when the initial carbon source is exhausted. This nitrogen supply is considered important in production phase when cells are first exposed to the precursor compound feedstock, such as ethyl laurate. Production phase is split into an early production phase, or induction phase, and a main production phase.

In an embodiment, early production phase is initiated upon exhaustion of the initial carbon source such as dextrose. Only after exhaustion of the initial carbon source the cells are first exposed to the fatty acid which induces a number of genes required for bioconversion of the fatty acid to the dicarboxylic acid. The yeast cells are also provided with a constant feed of dextrose to supply carbon and energy for the induction process and for maintaining cell viability. Dextrose feed rate preferably does not change between early and main production phases. Substrate feed of the precursor compound such as ethyl laurate (or a mixture of ethyl laurate, lauric acid and ethanol) is initiated at the dissolved oxygen (DO) spike that signals exhaustion of the initial carbon source. Exposure of the cells to this new carbon source induces expression of genes in the omega-oxidation pathway among other important genes. The substrate feed rate is low during this phase since lauric acid is somewhat toxic and accumulates until induction is complete. Complete induction generally takes about 6 hours, although the low feed rate is maintained for 24 h. As cells are producing new enzymes for bio-conversion, a ready supply of nitrogen is required, and so the media is formulated with excess ammonium sulfate to provide a source of nitrogen after growth phase. Along with initiation of the precursor compound substrate feed, a co-substrate feed of dextrose is initiated. This co-substrate is required in those embodiments, where the microorganism strain is completely blocked in beta-oxidation. Since the microorganism cannot derive any energy from the fatty acid substrate, the co-substrate provides energy required to perform bioconversion of the substrate to DDDA.

In an embodiment, the main production phase and thus the bioconversion step begins by increasing substrate feed rate of the precursor compound. It is important that the targeted substrate feed rate is established as quickly as possible. Substrate feed rates lower than target rate will result in lower dicarboxylic acid productivity. A substrate feed rate higher than the target exceeds the culture's ability to form dicarboxylic acid and results, for example and in case ethyl laurate is used as precursor compound, in accumulation of lauric acid which is much more toxic than the product, i.e. DDDA. Accumulation of lauric acid also reduces yield and purity of product in broth and complicates the purification process. Since lauric acid is a chain terminator in polymer synthesis, its removal from the final product is critical with regard to the use of the dicarboxylic acid and DDDA in particular in polymerization. In an embodiment, lauric acid is removed during thin-film evaporation, preferably during thin-fil evaporation with subsequent distillation.

In an embodiment, off-gas data is a very important tool in evaluating the "health" of the biocatalyst carrying out the bioconversion step, and is useful in matching the ethyl laurate feed rate to the level of cellular activity present. Typically, oxygen uptake rate (OUR) correlates strongly to health of the biocatalyst and dicarboxylic acid production rate, and can indicate precursor compound over-feeding.

In an embodiment, at the end of fermentation step and the bioconversion step the broth contains the desired dicarboxylic acid such as DDDA, cells, remaining media salts, and a mixture of feed components and by-products. Typically coming in with the ethyl laurate feed is a mixture of fatty acids, such as saturated and unsaturated C16 (palmitic acid and palmitoleic acid) and C18 (stearic acid and oleic acid) fatty acids, and small amounts of shorter chain saturated fatty acids (e.g. C10, C14) along with a variety of unknown compounds. In an embodiment, the strain will convert these fatty acids to their di-acid form, yielding measurable amounts of C10, C14, C16, and C18 dicarboxylic acids. An intermediate in the bioconversion is hydroxy-fatty acid such as hydroxylauric acid. Because of this, measurable quantities of corresponding hydroxy-fatty acids may be present and, respectively, detected. In an embodiment, of all the impurities, lauric acid and hydroxylauric acid are the most prevalent.

The present invention is related in an eighth aspect to a method for purifying a dicarboxylic acid from a medium, wherein the method comprises a nano-diafiltration step as disclosed and described herein, in particular disclosed and described herein in connection with the first aspect of the present invention. The dicarboxylic acid is preferably the one disclosed and described herein in connection with the first aspect. In such method for purifying a dicarboxylic acid, the starting material for the purification is preferably a medium containing the dicarboxylic acid. In an embodiment, the medium is an aqueous medium. In another embodiment, the medium is a medium obtained from a bioconversion step, preferably a bioconversion step as disclosed or described herein, more preferably in connection with the first and/or the second aspect. The medium obtained from a bioconversion step is preferably one from which any biomass used as a biocatalyst for the conversion of the dicarboxylic acid from a precursor compound, has been removed. In a preferred embodiment, the biocatalyst-free medium has been subjected to an ultrafiltration step, preferably an ultrafiltration step disclosed or described herein, more preferably in connection with the first and/or second aspect.

The present invention is related in a ninth aspect to a method for purifying a dicarboxylic acid from a medium, wherein the method comprises a distillation step as disclosed and described herein, in particular disclosed and described herein in connection with the second aspect of the present invention. The dicarboxylic acid is preferably the one disclosed and described herein in connection with the second aspect. In such method for purifying a dicarboxylic acid, the starting material for the purification is preferably a medium containing the dicarboxylic acid. In an embodiment, the medium is an aqueous medium. In another embodiment, the medium is a medium obtained from a bioconversion step, preferably a bioconversion step as disclosed or described herein, more preferably in connection with the first and/or the second aspect. The medium obtained from a bioconversion step is preferably one from which any biomass used as a biocatalyst for the conversion of the dicarboxylic acid from a precursor compound, has been removed. In a preferred embodiment, the biocatalyst-free medium has been subjected to an ultrafiltration step, preferably an ultrafiltration step disclosed or described herein, more preferably in connection with the first and/or second aspect. In another embodiment, the dicarboxylic acid is present as a precipitated dicarboxylic acid, preferably such precipitated dicarboxylic acid has been precipitated in an acidification step as disclosed and described herein. In a preferred embodiment thereof, the dicarboxylic acid has been precipitated from the afore-described medium.

It is within the present invention in its various aspects, including any embodiment thereof, that the nano-diafiltration step can be a nano-filtration step.

It will be acknowledged by a person skilled in the art that a middle section of a rectification column is the section of a rectification column which is arranged between the upper section and the lower section of a rectification column.

It is within the present invention that any indicated percentage (%) is % (wt/wt) unless explicitly indicated to the contrary.

It will be appreciated by a person skilled in the art that any process parameter disclosed herein such as temperature, pressure, pH and/or site of introduction into any device is applicable to each and any dicarboxylic acid to be produced in accordance with the present invention in its various aspects, including any embodiment thereof. It will also be appreciated by a person skilled in the art that any process parameter disclosed herein such as temperature, pressure, pH and/or site of introduction into any device is applicable in particular to dodecanedioic acid (DDDA) which is produced, to be produced or purified in accordance with the present invention in its various aspect, including any embodiment thereof.

It is to be acknowledged that the terms "method for the production of a dicarboxylic acid" and "method for producing a dicarboxylic acid" are used interchangeably herein.

It is to be acknowledged that any reference to any "embodiment of the invention" or to "embodiment" is a reference to any aspect of the present invention as disclosed herein, including any embodiment thereof.

It is within the present invention that any embodiment of any aspect of the present invention as disclosed herein is also an embodiment of each and any other aspect of the present invention.

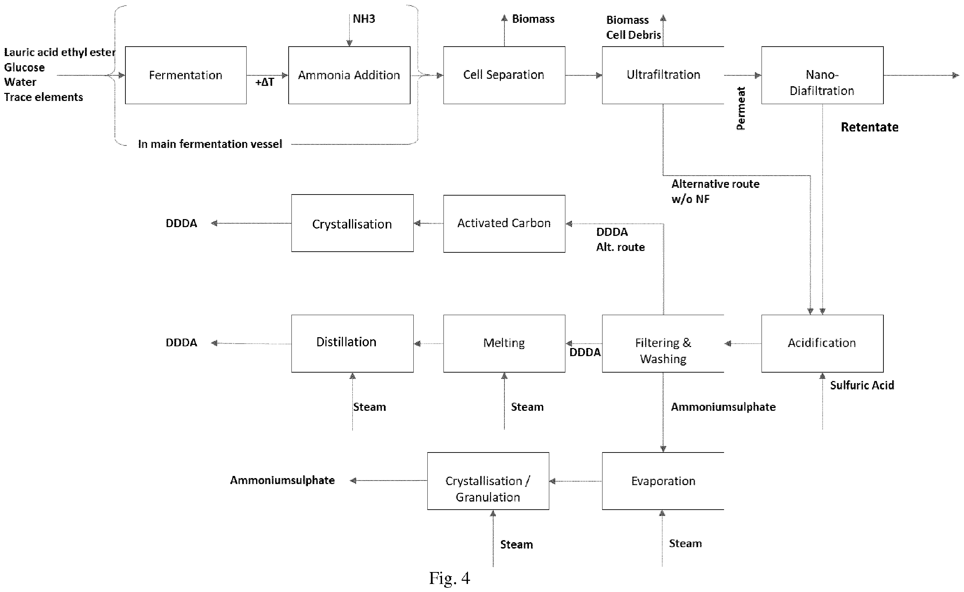

In an embodiment of the method for producing a dicarboxylic acid such as DDDA according to the first and the second aspect, the method comprises the steps illustrated in Fig. 4.

The present invention is now further illustrated by the following Figs. from which further features, embodiments and advantages of the present invention may be taken. More specifically,
Fig. 1 shows a block flow diagram for an acidification and a precipitation step;
Fig. 2 shows a flow diagram for the treatment of raw DDDA in a thin film evaporator and subsequent treatment in a rectification column;
Fig. 3 shows a block flow diagram of the fermentation step; and
Fig. 4 shows a block flow diagram of an embodiment of the method for producing DDDA according to the first and second aspect.

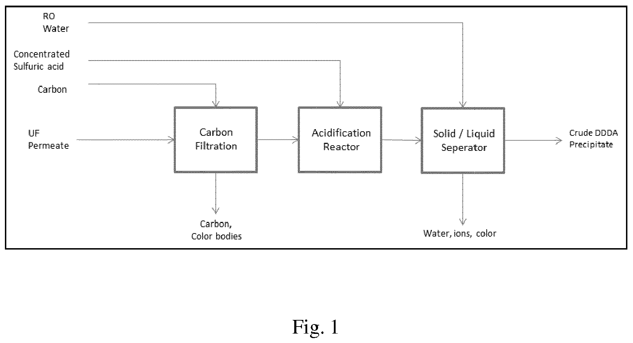

Fig. 1 shows a block flow diagram for an acidification and a precipitation step. In an embodiment of the method of the first aspect and the second aspect of the invention, nano-diafiltration retentate is added to an agitated vessel and mixed with activated carbon and filter aid. The solution is heated to about 60°C and mixed for 10 min. The mixture is then filtered through a 30µm polypropylene filter to remove the activated carbon. This activated carbon filtration step uses an activated carbon to DDDA mass ratio of about 1:10, whereby other ratios may be used and determined, respectively, by routine tests.

The filtered solution is then heated to 96 - 100°C. Sulfuric acid (98% (wt/wt)) is added and mixed with the nano-diafiltration retentate to achieve a pH of 1.6 - 1.8 at 100°C. The sulfuric acid is charged at a continuous rate over a two-hour period. The final amount of concentrated sulfuric acid fed is about 4% volume sulfuric acid / volume of charged filtrate obtained from the ultrafiltration step. During acid addition the product precipitates out of solution. Once acid addition is complete, the solution is held at a temperature of about 90°C for 30 minutes. Then the solution is cooled to 25 - 30°C. The precipitated DDDA is separated via an isolation device and washed with de-ionized water. The purpose of this step is to remove ions, such as those from media components, base additions, and sulfate from the acidification step. The preferred technology is a pusher centrifuge, belt filter or rotary vacuum filter capable of washing while isolating a dewatered cake.

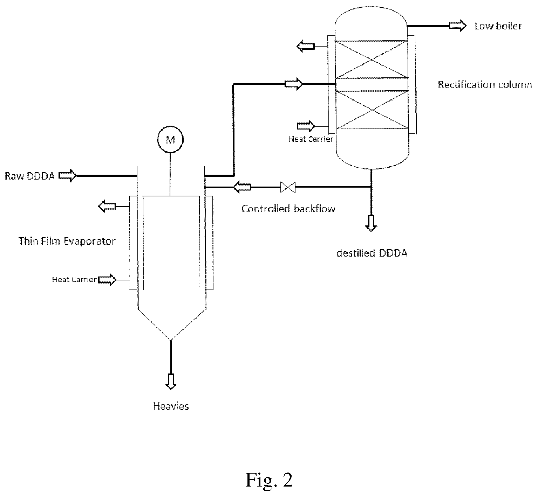

Fig. 2 shows a diagram for the treatment of raw DDDA in a thin film evaporator and subsequent treatment in a rectification column.

Liquid DDDA at a temperature of 140 °C is directed to a thin film evaporator and vaporized there. Evaporation temperature is preferably about 190 °C at 1 mbar so as to provide optimum separation. The high boilers (referred to as "heavies" in Fig. 2) do not change to the gas phases at this temperature and are removed from the thin-film evaporator, for example by wipers. The conditions for evaporation range from 190°C at 1 mbar to about 240°C at 10 mbar.

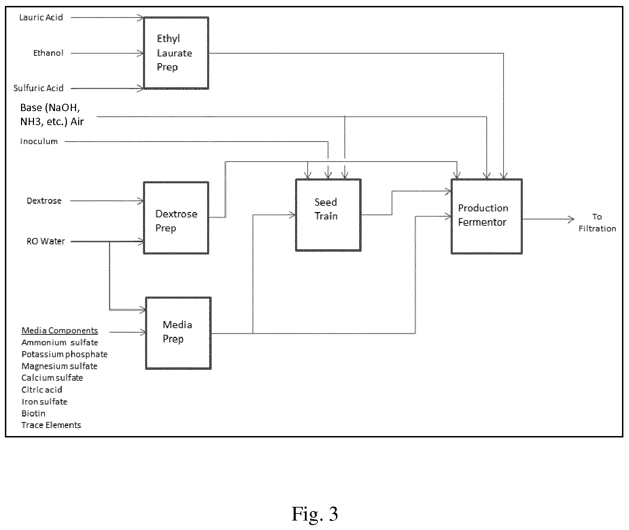

Fig. 3 shows a block flow diagram of the fermentation and bioconversion part of an embodiment of the method for the production process of a dicarboxylic acids of the present invention. In a first step, the precursor compound for the dicarboxylic acid production is produced, in the case of dodecanedioc acid (DDDA) this is ethyl laurate. Ethyl laurate is produced in an esterification reaction from lauric acid and ethanol in the presence of sulfuric acid and subsequently distilled to high purity. This step is called "Ethyl Laurate Prep" in Fig. 3 and depicted in the upper line of Fig.3; this step may be carried out either internally or by a supplier of ethyl laurate.

In the lower part of Fig. 3, the preparation of fermentation medium is depicted, whereby the components listed are mixed into water and passed through a sterile filter before they enter the seed train or the main fermentor. In the middle part, dextrose solution is prepared and such dextrose solution, also after passage through sterile filtration or heat sterilization, enters the seed train or main production fermentor. The seed train consists of several consecutive fermenters of different sizes and is used to cultivate the microorganism capable of producing the dicarboxylic acid carrying out, and respectively, of carrying out the bioconversion step. After the seed culture has been transferred to the production fermentor the microorganism used as a biocatalyst is cultivated to a cell density sufficient to carry out bioconversion, typically 3 to 4 days of cultivation time. Then the precursor is continuously added to the production fermenter under continuous aeration and addition of medium and stirring. The biocatalyst is converting the precursor compound to the dicarboxylic acid, in case of ethyl laurate dodecanedioic acid is formed. After a period of 1 to 2 days all precursor is converted to product and small amounts of byproduct. The bioconversion broth is then directed to the downstream and purification processes further disclosed herein.

Vaporized DDDA is directed at the same temperature and pressure to a rectification column, preferably directed to half the height of the rectification column, and separated by means of the trays from low-boilers such as fatty acids, lactams lactones and/or short dicarboxylic acids. In an embodiment, the rectification comprises at least eight theoretical trays. Distilled liquid DDDA is collected at the bottom of the rectification column.

Fig. 4 shows a block diagram of an embodiment of the method for the production of DDDA according to the first and the second aspect.

In a fermentation vessel, a microorganism such as yeast Candida vishvanathii is grown to reach the biomass necessary to for the bioconversion step. In the bioconversion step, paraffin or fatty acids used as a precursor compound is converted to the dicarboxylic acid, which is subsequently treated in the same vessel with ammonia after finishing bioconversion. The dicarboxylic acid is then transformed into the ammonia salt of the acid and therefore soluble in the fermentation broth. The biomass is subsequently separated by means of a centrifuge and fermentation broth is fed to an ultrafiltration unit to remove final remains of biomass and cell debris. The now clarified broth is nano filtrated where the diammonium salt of the dicarboxylic acid is collected on the retentate side of the nano filtration. As the nano filtration is working as dia-filtration the major part of the impurities with low molecular weight is washed out and leaves the ammonium salt of the dicarboxylic acid more purified. As an alternative, the nano filtration can be bypassed, but in such case the broth contains more impurities in comparison to the nano filtrated material. In the next step, the dicarboxylic acid is precipitated by addition of sulfuric acid, as the solubility of the dicarboxylic acid is very low. The precipitate is then filtered or alternatively centrifuged from the ammonium sulphate solution, washed with cold water and fed to a fluidized bed dryer unit. The ammonium sulphate solution is concentrated to 40% dry material and either crystallized or granulated. The dried dicarboxylic acid can be purified by two different methods. One way is to melt the dicarboxylic acid in a melting vessel and feed the dicarboxylic acid to a thin-film evaporation unit. In the thin-film evaporation unit, the dicarboxylic acid is evaporated on the wiped heating wall of the evaporation column and the vapour is brought to mid tray of a rectification column. The distilled dicarboxylic acid is then taken from the bottom of the rectification column. The alternative way to purify the dicarboxylic acid is to resolve the dicarboxylic acid in water or an organic solution such as acetic acid. The dicarboxylic acid solution is treated with heat and activated carbon to remove impurities and colour bodies. After removal of the activated carbon the solution is cooled down and the purified dicarboxylic acid precipitates. The crystalline dicarboxylic acid will be washed with cold water and dried.

## Claims

1. A method for the production of a dicarboxylic acid, wherein the method comprises
- a bioconversion step, wherein in the bioconversion step, the dicarboxylic acid is produced from a precursor compound contained in a medium; and a
- purification step for purifying the dicarboxylic acid from the medium, wherein the purification step comprises a nano-diafiltration step and/or a distillation step, wherein preferably if the purification step comprises both the nano-diafiltration step and the distillation step, the nano-diafiltration step is carried out prior to the distillation step, and
wherein the dicarboxylic acid is selected from the group comprising decanedioic acid, dodecanedioic acid, tetradecanedioic acid and hexadecanedioic acid, preferably the dicarboxylic acid is dodecanedioic acid (DDDA).

2. The method of claim 1, wherein if the purification step comprises the nano-diafiltration step, the medium containing the dicarboxylic acid is subjected to the nano-diafiltration step, wherein the retentate of the nano-diafiltration contains the dicarboxylic acid.

3. The method of any one of claims 1 to 2, wherein the membrane used in the nano-diafiltration step has a cut-off value of between 150 Da and 300 Da, preferably the cut-off value is ≤ 150 Da.

4. The method of any one of claims 1 to 3, wherein the dicarboxylic acid containing retentate of the nano-diafiltration step is subjected to an acidification step, preferably in the acidification step, sulfuric acid is added to the dicarboxylic acid containing retentate of the nano-diafiltration step and the dicarboxylic acid is precipitated from the retentate, and the precipitated dicarboxylic acid is optionally washed.

5. The method of claim 1, wherein the method does not comprise the nano-diafiltration step, and wherein the dicarboxylic acid obtained from the bioconversion step is subjected to the distillation step.

6. The method of claim 5, wherein the dicarboxylic acid obtained from the bioconversion step or from a stage of the purification step carried out prior to the distillation step is obtained as precipitated dicarboxylic acid, preferably the dicarboxylic acid is obtained as precipitated dicarboxylic acid from an acidification step.

7. The method of any one of claims 4 and 6, wherein the precipitated dicarboxylic acid is subjected to a melting step, wherein in the melting step, the precipitated dicarboxylic acid is melted, preferably at a temperature of about 140 °C and subjected to the distillation step.

8. The method of claim 7, wherein in the distillation step, the melted dicarboxylic acid is heated so as to obtain vaporized dicarboxylic acid, preferably the melted dicarboxylic acid is heated in a distillation column so as to obtain vaporized dicarboxylic acid.

9. The method of claim 8, wherein the conditions for vaporization of the dicarboxylic acid ranges from 190°C at 1 hPa to about 240°C at 10 hPa.

10. The method of any one of claims 8 to 9, wherein the dicarboxylic acid is vaporized in a thin-film evaporator, wherein, preferably in the thin-film evaporator a heavy-boiler is separated from the dicarboxylic acid, more preferably the dicarboxylic acid obtained from the thin-film evaporator is conducted to a rectification column, wherein, preferably, in the rectification column the dicarboxylic acid is separated from the low-boiler, more preferably the dicarboxylic acid is introduced to a feed tray at the middle section of the rectification column.

11. The method of any one of claims 1 to 4, wherein the method does not comprise the distillation step and wherein the precipitated dicarboxylic acid is dissolved in a fluid, preferably the fluid is water, an organic solvent or a mixture of water and an organic solvent.

12. The method of claim 11, wherein the fluid containing the dicarboxylic acid is subjected to a crystallization step, wherein the dicarboxylic acid is crystallized from the dicarboxylic acid containing fluid in the crystallization step, whereupon crystallized dicarboxylic acid and a supernatant are provided, preferably the crystallized dicarboxylic acid is removed from the supernatant, preferably by centrifugation or filtration.

13. The method of claim 12, wherein the crystallized dicarboxylic acid removed from the supernatant is subject to a washing step and/or drying step.

14. The method of any one of claims 1 to 13, wherein
(a) the precursor compound comprises or is an ethyl ester or methyl ester of the monocarboxylic acid of the dicarboxylic acid to be produced, preferably the precursor is or comprises
an ethyl ester or methyl ester, preferably an ethyl ester, of decanoic acid in case the dicarboxylic acid to be produced is decanedioic acid;
an ethyl ester or methyl ester, preferably an ethyl ester, of dodecanoic acid in case the dicarboxylic acid to be produced is dodecanedioic acid;
an ethyl ester or methyl ester, preferably an ethyl ester, of tetradecanoic acid in case the dicarboxylic acid to be produced is tetradecanedioic acid; and
an ethyl ester or methyl ester, preferably an ethyl ester, of hexadecanoic acid in case the dicarboxylic acid to be produced is hexadecanedioic acid; and/or
(b) the precursor compound comprises or is an alkane compound, wherein preferably the alkane compound is selected from the group comprising decane, dodecane, tetradecane and hexadecane, more preferably the precursor compound is or comprises
decane in case the dicarboxylic acid to be produced is decanedioic acid;
dodecane in case the dicarboxylic acid to be produced is dodecanedioic acid;
tetradecane in case the dicarboxylic acid to be produced is tetradecanedioic acid; and
hexadecane in case the dicarboxylic acid to be produced is tetradecanedioic acid.

15. The method of any one of claims 1 to 14, wherein in the bioconversion step bioconversion of the precursor compound is effected by a biocatalyst, preferably the biocatalyst is a microorganism and more preferably the biocatalyst is a yeast.
